# EUROPEAN PATENT APPLICATION

(11) **EP 3 912 648 A1**
(43) Date of publication of application: **24.11.2021**
(21) Application number: 20175890.1
(22) Date of filing: 21.05.2020
(51) Int. Cl.: A61L 2/20, A61L 2/10, A61L 2/08, A61L 2/04, A61L 2/24, A61L 2/26, B01D 53/047

(54) **METHOD AND CORRESPONDING APPARATUS FOR STERILIZATION IN THE MEDICAL FIELD**

(71) Applicant: Padrin, Stefano, 28060 Cureggio (NO) (IT)
(72) Inventor: Padrin, Stefano, 28060 Cureggio (NO) (IT)
(74) Representative: Botti, Mario

(57) **Abstract**

The present disclosure relates to a method and corresponding apparatus for sterilization in the medical field of objects, instruments and/or items that are potentially contaminated and to be sanitized.

The method includes the following steps:
- Intaking ambient air into a sterilization device;
- concentrating the oxygen present in the air;
- generating ozone from concentrated oxygen;
- introducing ozone into a sterilization chamber (15) wherein said objects (A) are received;
- applying UV rays for a UV generator (17) in said sterilization chamber;
- treating the atmosphere of said sterilization chamber in a decontamination chamber wherein ozone is decomposed;
- reintroducing the decontaminated atmosphere into the environment.

Advantageously, the ultraviolet rays are low-wavelength germicides and in said sterilization chamber infrared rays are also applied.

## Description

### Field of application

The present invention relates to a method for sterilization in the medical field of objects, instruments and/or items that are potentially contaminated and to be sanitized.

The invention also relates to an apparatus made and operating according to the above method.

### Prior art

As it is well known in this specific technical field, there is often the need to sterilize various objects, instruments, items and/or devices used in the medical field and that may have been potentially contaminated.

We particularly refer to objects, instruments, items and/or devices that must be reused; i.e. not having disposable features.

The use of sterilization devices is already known in the art. By way of non-limiting example, generally the medical facilities sterilize objects and instruments to be reused by means of autoclaves with pressurized steam and a superheated water process. This process is commonly used in microbiology, medicine, body piercing, veterinary science, dentistry, podiatry and metallurgy. Autoclaves are also used for the care of carbon fiber composite parts and rubber parts and also for the treatment and sterilization of some hospital waste.

However, this steam sterilization process requires many steps and resources. Indeed, a typical sterilization procedure that uses an autoclave requires distilled water, bags for sterilization or biological risk, washing with germicidal liquid spray, ultrasonic bath and drying with air compressors.

The drawbacks of these autoclaves include a high energy consumption, waste of time due to multi-stage disinfection sequences, germicidal chemicals that are toxic to the environment. There are also safety risks associated with high power and high pressure machinery.

There are also other intrinsic limitations of this known technology since moist heat is used and heat labile products, such as some plastic or paper materials, cannot be sterilized in this way.

What's more, steam autoclave sterilization, even at high temperatures, is not always effective to prevent the transmission of viroids through medical and surgical equipment.

The prior art also suggests the UV sterilization, which is used in the purification and disinfection of water, air and/or surfaces. Over the years, ultraviolet technology has established itself as method of choice for effectiveness, economy, safety, speed, ease of use and because the process is free of by-products.

This UV sterilization technique is rapid and does not require the use of heat or chemicals. However, up to date, this process has not been reduced in practice to devices and methods that are readily accepted for common use.

Although advantageous under various aspects and substantially meeting the purpose, the UV ray sterilizers currently on the market are not adequately configured for portable use and/or for equipment that can be used as sterilization units of the commercial medical type, therefore likely to replace the conventional autoclaves or capable of acting as low-cost portable home units.

The object of the present invention is to conceive a method and corresponding apparatus having respective functional and structural features, such as to allow providing a structure that is configured to quickly sterilize and sanitize objects, instruments, items and/or devices used in the medical field and likely to be reused.

Another object of the invention is to provide an apparatus of the above type that is suitable for use in sterilization of plastics or materials having a delicate a composite structure that is not suitable for autoclave sterilization and not having disposable features.

A further object of the invention is to provide an apparatus of the above type and easy to use, effective and at low cost.

Finally, a further object of the invention is to provide an apparatus having a portable structure and easy to install in any medical practice that needs to effectively and frequently sterilize everyday objects and instruments.

### Summary of the invention

The solution idea underlying the present invention is to combine a unique portable sterilization apparatus with the use of UV rays and ozone but providing a further final decomposition treatment of ozone to reintroduce oxygen into the ambient air.

Based on this solution idea, the technical problem is solved by a sterilization method of objects, instruments and/or items that are potentially contaminated and to be sanitized, comprising:
- intaking ambient air into a sterilization apparatus;
- concentrating the oxygen present in the air;
- generating ozone from concentrated oxygen;
- introducing ozone into a sterilization chamber wherein said objects are received;
- applying UV rays in said sterilization chamber;
- treating the atmosphere of said sterilization chamber in a decontamination chamber wherein ozone is decomposed;
- reintroducing the decontaminated atmosphere into the environment.

Advantageously, in said sterilization chamber infrared rays are also applied.

Furthermore, said UV ultraviolet rays are low-wavelength germicides.

Advantageously, the step of concentrating oxygen and generating ozone is activated concurrently with the outside air intake.

Furthermore, the step of applying the UV rays and of emitting ozone are simultaneously activated.

The invention also relates to an apparatus for sterilization of objects (A), instruments and/or items that are potentially contaminated and to be sanitized, comprising:
- an oxygen concentrator receiving inletting ambient air;
- an ozone generator downstream of said concentrator;
- a sterilization chamber wherein said objects are received and whereinto ozone is made to flow;
- means for generating UV ultraviolet rays in said sterilization chamber;
- a decontamination chamber for decomposing into oxygen the ozone downstream of the sterilization treatment in said sterilization chamber.

The apparatus according to the invention further comprises an infrared rays-generator that is associated with the sterilization chamber.

Furthermore, a supervision and control electronic unit that is active on said concentrator and ozone generator, as well as for piloting said means for generating UV ultraviolet rays which are interlocked to temperature, operating pressure and ozone (O₃) concentration sensors, is provided.

Such an apparatus is configured with a storage compartment or tank container for the objects to be sanitized that is closed by a watertight lid which offers access to the sterilization chamber.

The lid is provided with lateral telescopic arms, having a pre-fixed friction, which allow angularly keeping it raised during the step of inserting and loading the objects and items to be sterilized and sanitized.

Advantageously, the perimeter edge of the storage compartment and of the lid provides for electromagnetic closure means and counter-means of the sterilization chamber; said means are interlocked to the supervision and control electronic unit by means of a frontal control and display panel of the touch screen type that is available to the user.

The features and advantages of the method and apparatus of the present disclosure will become apparent from the following detailed description of a respective embodiment given by way of non-limiting example with reference to the appended drawings.

### Brief description of the drawings

- Figure 1 shows a schematic block view of the steps of the method of the present disclosure;
- Figure 2 shows a schematic block view of the component parts of an apparatus made according to the method of the present invention;
- Figure 3 shows a functional block diagram that schematically illustrates the method of the present invention carried out in the apparatus of Figure 2;
- Figure 4 shows a perspective and schematic view of a portable apparatus that is likely to operate according to the method of the present invention;
- Figures 5 and 6 show respective perspective and schematic views in vertical elevation of the apparatus of Figure 3 in two different operating conditions;
- Figure 7 shows a schematic view of the ozone-generation phenomenon starting from oxygen between two plates of a capacitor;
- Figure 8 is a schematic view of a portion of the electromagnetic wave frequency spectrum in which the UV-C rays are highlighted.

### Detailed description

With reference to these figures, reference number 1 globally and schematically represents a method according to the present disclosure for sterilization of objects, instruments and/or items that are potentially contaminated and to be sanitized.

The concerned objects may be of any type, though the method of the present invention was particularly conceived to sanitize objects, instruments, items and/or devices used in the medical field.

The invention is particularly suitable for sterilization and sanitation of objects that are supplied to healthcare personnel, such as for instance: masks, protective gloves, gowns and protective suits, footwear, headphones, protective glasses and visors. Just by way of example, these objects are generically indicated in the figures with letter A.

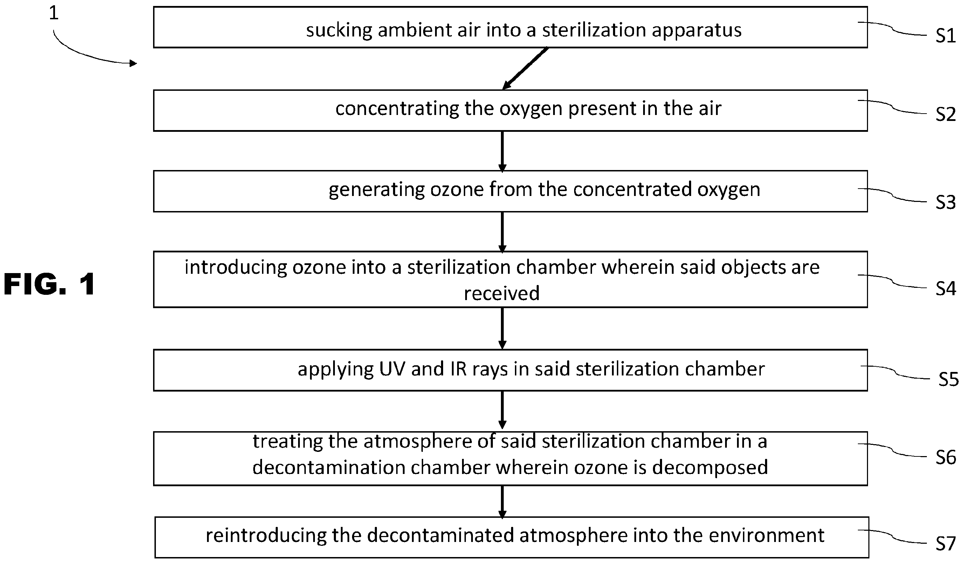

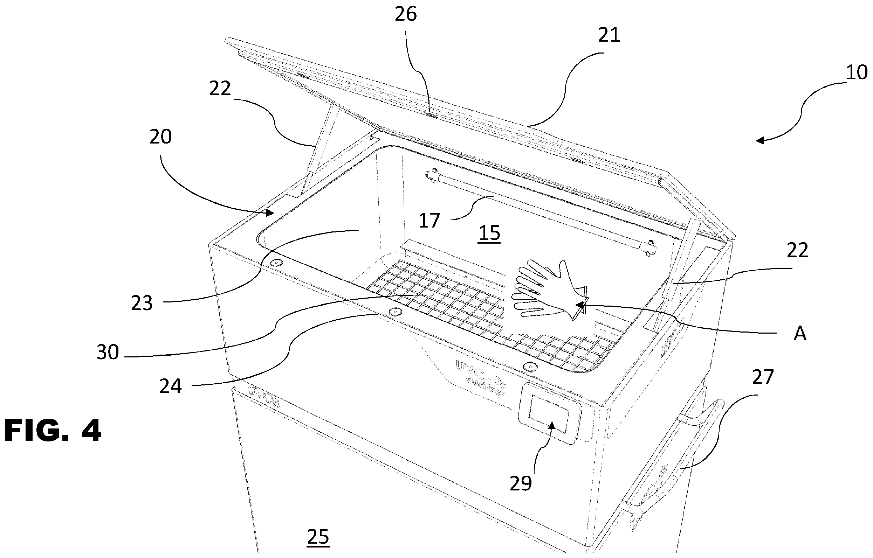

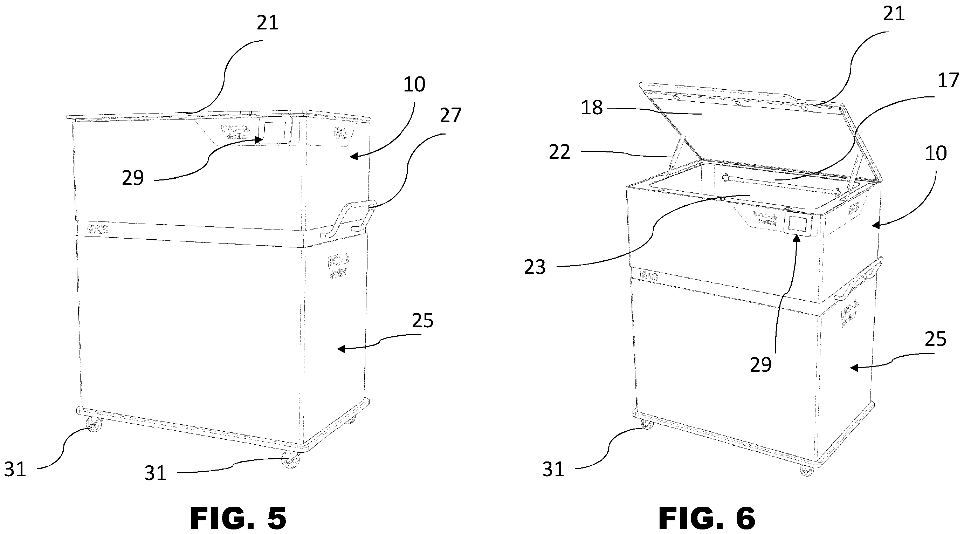

The method of the present invention is carried out in an apparatus 10 that is illustrated in figures 4, 5 and 6 and includes a sequence of steps that will be illustrated hereinafter with particular reference to the example of figure 1.

A first step S1 of the method provides for intaking ambient air into a sterilization apparatus, for instance the above apparatus 10.

This step has the advantage of using ambient air as primary source of oxygen supply.

In a successive step S2, the oxygen present in the air is concentrated so as to obtain a percentage of O₂ that is enough for a successive treatment.

At this point, in step S3, ozone O₃ is generated from oxygen O₂ that was concentrated in step S2.

Obviously, a skilled person could provide for a specific ozone O₃ adduction to by-pass steps S2 and S3; however, this possible option is relatively more expensive, and the method of the present invention provides for obtaining the ozone necessary for the actual sterilization step directly from the adduction of ambient air.

The following step S4 provides for introducing ozone into a sterilization chamber wherein the objects to be sterilized and sanitized are collected. The sterilization chamber is part of the apparatus 10 according to the invention and will be described hereinafter in greater detail.

Ozone has been recognized by the ministry of health (protocol no. 24482 of July 31, 1996) as a natural aid for the sterilization of environments contaminated by bacteria, viruses, spores. Ozone kills 99.999% of airborne pathogens, including SARS Coronavirus and influenzas such as H5N1. A specific study on the effectiveness of ozone on the new Coronavirus is currently underway in China, at the Hubei Institute of Virology.

Thanks to its virucidal ability, ozone offers the possibility to disinfect hardly accessible spaces.

Advantageously, according to the invention, UV rays are applied in said sterilization chamber in combination with ozone. This step is schematized by block S5 of figure 1.

More particularly, germicidal UVC rays are used, i.e. UV having a short wavelength.

Germicidal ultraviolet radiation is a sterilization method that uses ultraviolet light (UV) with wavelengths included for instance in the UV-C band (between 280 and 100 nanometers), which modifies the DNA or RNA microorganisms and therefore prevents them from reproducing or being harmful.

Furthermore, still according to the present invention, in the sterilization chamber infrared rays are also applied, which enhance the sanitizing effect of the UV rays.

The UVC waves act on the surfaces of the objects to be sterilized, thus ensuring a total antiviral action. Moreover, the UVC waves used together with ozone O₃ allow reducing the oxidizing power of ozone O₃ itself.

The cycle performed with ozone O₃ allows reaching the innermost parts of objects, even those having complex shapes.

Moreover, the infrared rays IR complete the germicidal action by raising the temperature inside the sterilization compartment.

However, at the end of the sterilization process a high ozone concentration is present inside the sterilization chamber. Ozone is harmful to human health, but the method of the present invention also allows solving this potential problem.

The steps of the method according to the present invention continue with step S6 of treating the atmosphere of said sterilization chamber. Basically, the atmosphere present in the sterilization chamber at the end of the treatment with ozone, and at the end of the application of UV and infrared rays, is treated in a decontamination chamber wherein ozone is decomposed.

This passage allows obtaining again oxygen O₂ and reintroducing into the environment an ozone-free atmosphere.

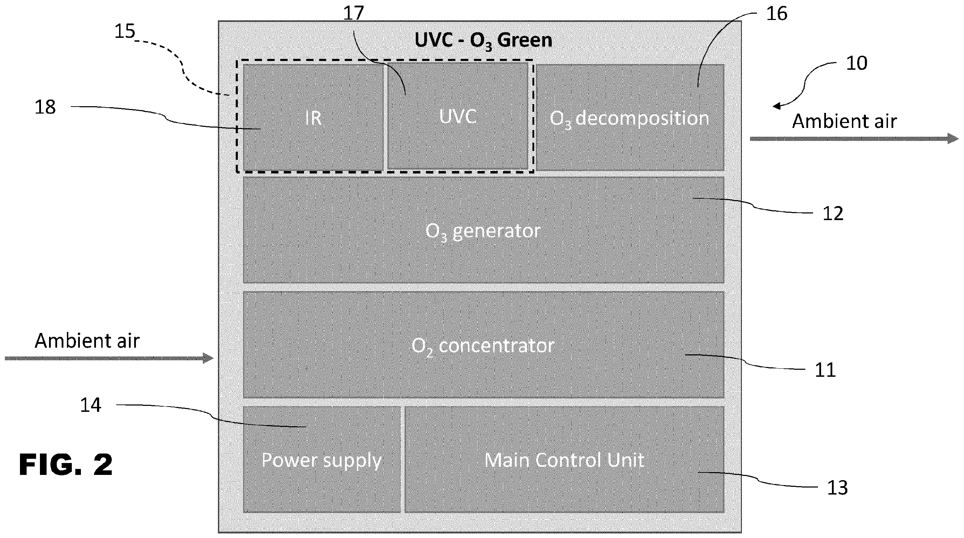

Figure 2 shows a schematic view of the component parts of the apparatus 10 according to the present invention.

An oxygen concentrator 11, which operates in cooperation with an ozone generator 12, is provided.

A UVC-ray generator 17 and an IR-ray generator 18 are associated with a sterilization chamber 15.

An ozone decomposition chamber 16 is also provided.

The entire apparatus is controlled and adjusted by a supervision and control electronic unit 13, whereas the component parts having an electronic operation are powered by a power supply unit 14.

Various sensors, globally indicated with reference number 19, which will be described hereinafter, and which are responsible for sending control signals to the control unit 13, are also provided.

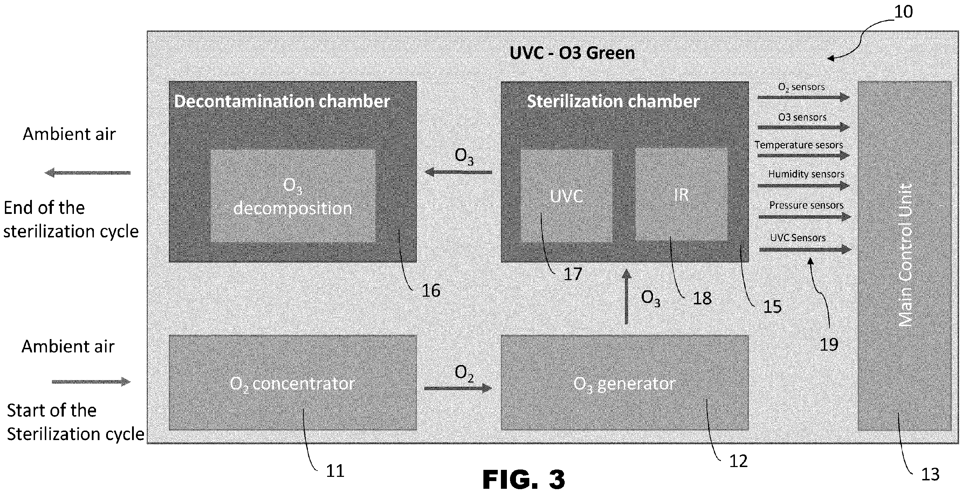

More in detail, with particular reference to the example of figure 3, the apparatus 10 draws inletting air from the surrounding environment at each start of the sterilization cycle.

This ambient air is treated in an oxygen concentrator 11.

The oxygen concentrator 11 is a device that concentrates the oxygen coming from a specific source, typically ambient air, providing an oxygen enriched gas mixture. The oxygen concentrator 11 uses a technology known as "pressure swing adsorption", i.e. a physical process for separating gas mixtures by adsorption under pressure.

The concentrator 11 works based on the principle of rapid adsorption of nitrogen of the ambient air on a molecular sieve, for example of zeolite, due to a produced atmospheric pressure (PSA) variation. Nitrogen is subsequently released with the return of pressure to ambient values.

This type of adsorption system is basically a "filter" for nitrogen, which allows other atmospheric gases to pass the zeolite sieve without problems. Therefore, at the end of this process, highly concentrated oxygen remains as main residual gas.

The PSA technology is a reliable, fast, convenient and economic technique for generating oxygen in small or medium quantities. Porous zeolite, at high pressures, adsorbs large quantities of nitrogen, due to its large contact surface. Later, after the separation of oxygen and the other gases that are not adsorbed, through a further passage, nitrogen is de-adsorbed by the molecular sieve. The oxygen concentrator 11 is compact in structure and includes an air compressor, two cylindrical containers filled with zeolite pellets, a pressure equalization tank, and some valves and pipes.

An ozone generator 12 is provided downstream of the concentrator 11. For the production of ozone the oxygen produced by the oxygen concentrator 11 is used as explained above.

Due to its relatively very short radioactive half-life, ozone is always produced *in situ* by the generator 12. Advantageously, in the generator 12 the ozone production electric mode called corona effect or corona discharge has been adopted, which has some advantages. However, nothing prevents the adoption of an alternative mode such as UV-light without this implying any limitation on the Applicant's rights.

UV radiation is viable where the production of small quantities of ozone is required (for instance in laboratories).

The advantages obtained by using a crown-effect ozone generator 12 are as follows:
- the possibility of building generators of limited dimensions;
- being able to produce ozone by minimizing the production of other irritating gases;
- longevity of the crown-effect cells, which can exceed ten years;
- high productivity.

In the ozone generator 12 there is an element that is responsible for the corona discharge effect, which provides a capacitive load.

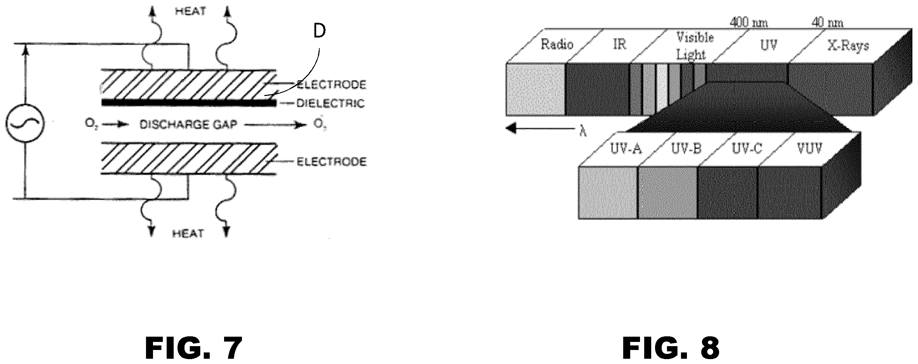

Figure 7 shows a rough diagram of the ozone generation phenomenon produced by oxygen as a direct result of the electric discharge. This electric discharge breaks down the stable oxygen molecule and forms two oxygen radicals. These radicals can be combined with the oxygen molecules to form ozone.

To control and maintain the electric discharge, there is a dielectric layer D, contained in ceramic or glass. Any excessive heat of the electrodes is dissipated by air.

The ozone O₃ produced by the generator 12 is transferred into the sterilization chamber 15 in which the objects and items to be sanitized have been previously accepted and collected. UVC and IR rays are also generated in this chamber 15.

UVC rays are generated by UV lamps in a UVC generator 17.

Science recognizes the bactericidal effects of the ultraviolet area of the electromagnetic spectrum.

The specific wavelengths that are responsible for this reaction are located between 240 and 280 nanometers (nm) with a peak at a wavelength of 265 nm and are known as UV-C.

Figure 8 schematically shows the UV-C rays within the electromagnetic wave spectrum.

The UVC band is effective in destroying bacteria, since its short wavelength is able to penetrate the cell walls of microorganisms.

When a microorganism is exposed to UV-C, the nuclei of the cells, due to the photovoltaic process, are modified to such an extent that cell division and therefore reproduction are inhibited.

The UVC generator 17 comprises a molten quartz tube. The inert gas with which the tube is filled in, provides the primary discharge and the necessary action to excite and vaporize the tiny mercury deposits therein contained.

A low pressure UV lamp is capable of producing lines between 185 and 254 nm.

Instead, as for the IR-ray generator 18 of the chamber 15, it includes IR lamps.

Infrared rays are electromagnetic waves whose wavelength is greater than 780 nanometers, which is a value that limits the passage from the spectrum of visible light to that of invisible light. The IR panels 18 are used to heat the objects inside the sterilization chamber 15.

Downstream of the sterilization chamber 15 a further treatment chamber 16 is provided, which we define as decontamination chamber. In this further chamber 16 the residual ozone of the sterilization process is advantageously decomposed. The decontamination chamber 16 may also spatially coincide with the sterilization chamber 15, but we can define it downstream since the decontamination process takes place temporally in a subsequent step to sterilization.

At the end of the sterilization cycle, a certain amount of ozone is always present in the sterilization chamber.

Despite the germicidal functions of ozone, it has been shown that in turn it causes various types of harmful effects on the respiratory tract:
- irritation of the respiratory system
- decreased lung function
- increase in asthmatic episodes and other respiratory diseases
- inflammation of the respiratory tract

The ozone level is lowered in the chamber 16 by means of a thermal process, by raising the temperature in a controlled manner inside a decontamination tank where the ozone present in the sterilization chamber is made to flow.

The final result of this operation is oxygen, which is again reintroduced into the air of the surrounding environment.

Now, with particular reference to the examples of figures 4 and the following, reference number 10 globally and schematically indicates the apparatus made according to the present invention to carry out the sterilization method in the medical field of objects, instruments and/or items that are potentially contaminated and to be sanitized.

The apparatus 10 is a sterilizer that is classifiable as a class I medical device for the sterilization of potentially contaminated objects A.

As previously seen with reference to the method of the present invention, the sterilizing action inside the device 10 is carried out through the use of:
- high intensity UVC radiation;
- infrared rays (IR);
- ozone (O₃) emission.

The apparatus 10 looks like a container 20 equipped at the top with a watertight closing lid 21.

The lid 21 is raised by means of lateral telescopic arms 22, having a predetermined friction, which allow keeping it angularly raised during the step of inserting and loading the objects and items to be sterilized and sanitized.

The lid 21 closes a substantially storage compartment 23 for receiving and collecting the objects to be sanitized and which includes the sterilization chamber 15.

On the perimeter edge of the compartment 23 and of the lid 21 means 24 and counter-means 26 for electromagnetic closing the sterilization chamber 15 are provided.

Inside the chamber 15 the UVC-rays generator 17 with a UV lamp is visible.

Inside the apparatus 10 the supervision and control electronic unit 13 that is accessible from outside by means of a frontal control and display panel 29 of the touch screen type.

On the bottom of the chamber 15 a reticle 30 for supporting the material to be sterilized is provided.

Internally, fixed to the cover 21, there is the IR-ray emission panel 18.

The oxygen concentration and ozone generation components 11 and 12 are not visible in the perspective figures since they are contained within the structure of the apparatus 10.

The outside air inlet and outlet openings are on the back of the apparatus 10.

A parallelepiped-shaped support structure 25 is also provided, which is mounted on wheels 31 and which allows easy movement of the apparatus 10 with the aid of a side handle 27.

Let us now see the operating principle of the apparatus 10.

The sterilization cycle begins with the commissioning of the oxygen concentrator 11 (O₂), whose task is to supply oxygen to the ozone generator 12. The oxygen concentration supplied by the concentrator is equal to 93%.

The ozone generator 12, through the crown effect principle, transforms ozone O₃ into the inletting oxygen O₂ received from the concentrator 11. The ozone O₃ produced by the ozone generator is introduced into the sterilization chamber 15, where the objects A to be sterilized are contained.

Ozone O₃ level, temperature, humidity, UVC irradiation and operating pressure are constantly monitored and kept under control through the use of appropriate redundant sensors.

In combination with the ozone generator 12, the UVC generator 17 is activated to reduce the oxidizing power of the ozone itself.

The IR emitters 18 are also activated, which allow to bring the temperature of the sterilization chamber to the desired temperature, based on the objects to be sterilized.

To indicate to the system which objects are there in the sterilization chamber, an appropriate sterilization program is selected via a user interface 29 equipped with a touch-screen.

The use of different types of sensors 19, for instance temperature, humidity, pressure, UVC, O₂ and O₃ sensors arranged close to or inside the sterilization and/or ozone decontamination chamber 15 or 16 allow controlling the cycles and their repetitiveness over time.

At the end of the sterilization process there is a high ozone concentration, which is harmful to the health of people.

Ozone takes time to return to its stable parent, oxygen. The sterilizer 10 is able to drastically reduce the residual ozone depletion time, releasing oxygen into the air.

Once the actual sterilization cycle has finished, through the decontamination tank 16 and the production of heat, in a few seconds ozone is transformed into oxygen.

At this point it is possible to access the objects A contained in the sterilization chamber and to start a new sterilization cycle.

From an operational point of view, it is possible to sum up the operating steps of the apparatus 10 according to the sequence of actions shown below:
- insertion of the objects to be sterilized in the compartment 23 and thus in the sterilization chamber 15;
- selection of the sterilization cycle through the panel 29;
- closure of the lid 21;
- start of the sterilization cycle through the touch panel 29;
- entry into operation of the oxygen O₂ concentrator 11 and of the ozone O₃ generator 12;
- air intake from the external environment, the oxygen present in the air is concentrated up to a concentration equal to 93% and passed to the O₃ generator;
- the ozone generator 12 receives the inletting oxygen and, through the crown effect principle, produces O₃, which is introduced into the sterilization chamber;
- the O₂ concentrator 11 and the O₃ generator 12 remain in operation for as long as necessary until the desired level of O₃ is reached in the sterilization chamber 15 by the selected program;
- the ozone O₃ level is constantly measured in the sterilization chamber 15 through sensors connected to the control unit 13;
- the UVC lamps 17 that reduce the oxidizing power of ozone and that also have germicidal power are activated;
- the IR emitters 18 are activated;
- the objects A inside the sterilization chamber 15 are heated to the temperature set by the program selected by the user and controlled by the temperature sensors;
- once the time necessary to sterilize the objects A inside the sterilization chamber 15 has ended, the ozone O₃ produced is poured into the decontamination tank 16 and a thermal process to decompose O₃ into O₂ is activated;
- at the end of the whole process of sanitization and ozone decomposition, air containing oxygen is released into the environment.

The combination of the three simultaneous sterilization, sanitization and oxygen-release actions makes the device 10 effective even with different materials such as plastic, metal, electronic components and commonly used objects, either synthetic or natural.

The sterilizer 10 of the present invention operates a clean cycle, which we can define "green" since at the beginning of the sterilization cycle the system takes air from the external environment and, at the end of the sterilization cycle, still releases clean air to the external environment.

Additionally, the sterilization cycle is completely repeatable in its characteristics, thanks to the set of sensors which the machine is equipped with.

The method and apparatus according to the invention solve the technical problem and achieve several advantages, which are herein below listed:
- effectiveness; the presence of UVC, O₃ and IR generators in a single instrument guarantees a rapid disinfection of bacteria and viruses with a level of 99.999%;
- speed; the sterilization cycles are extremely rapid thanks to the combined UVC, O₃ and IR action;
- 360° action; the internal coating is antibacterial and reflective: while the internal grid allows an efficient UVC and ozone circulation, the radiations bounce off the walls reaching even the innermost parts and the most complex shapes of the objects to be sterilized;
- versatility; multiple sterilization programs for an effective action on multiple materials and surfaces: synthetic fabrics, TNT, natural fabrics, plastic, steel, paper and derivatives, electronic components;
- robustness and capacity; the supporting structure is made of steel. The internal volume 800x500x300 mm allows sterilizing many units simultaneously or medium-sized objects;
- safety; the electromagnetic safety lock prevents accidental opening before completing the cycles. The outer surfaces are coated with antibacterial material to avoid contamination;
- ease of use; simply load the apparatus, close the door, select the desired sterilization program in connection to the load through the touch screen. Duration and residual cycle time are clearly indicated on the display;
- convenience and eco-compatibility; the apparatus of the present invention allows sterilizing material otherwise intended to be destroyed, such as disposable individual protection devices. The possibility of reuse thanks to sterilization increases the availability of PPE and allows a significant saving of economic resources. The disposing costs of contaminated material are eliminated, preserving the environment from the production of special waste.
- clean cycle; no element harmful to human health is released into the external environment.

## Claims

1. Method for sterilization of objects (A), instruments and/or items that are potentially contaminated and to be sanitized, comprising:
- intaking ambient air into a sterilization apparatus (10);
- concentrating oxygen (O₂) present in the air;
- generating ozone (O₃) from concentrated oxygen (O₂);
- introducing ozone (O₃) into a sterilization chamber (15) wherein said objects (A) are received;
- applying UV ultraviolet rays into said sterilization chamber (15);
- treating the atmosphere of said sterilization chamber (15) in a decontamination chamber (16) wherein ozone is decomposed (O₃);
- reintroducing the decontaminated atmosphere into the environment.

2. Method according to claim 1, **characterized in that** in said sterilization chamber (15) infrared rays (IR) are also applied.

3. Method according to claim 1, **characterized in that** said UV ultraviolet rays are low-wavelength germicides.

4. Method according to claim 1, **characterized in that** the step of oxygen concentration and ozone generation is activated concurrently with the outside air intake.

5. Method according to claim 1, **characterized in that** the steps of UV-rays application and ozone emission are activated simultaneously.

6. Method according to claim 2, **characterized in that** the IR-rays application is also activated in the decontamination chamber (16).

7. Apparatus (10) for sterilization of objects (A), instruments and/or items that are potentially contaminated and to be sanitized, comprising:
- an oxygen (O₂) concentrator (11) receiving inletting ambient air;
- an ozone (O₃) generator downstream of said concentrator (11);
- a sterilization chamber (15) wherein said objects (A) are received and whereinto ozone (O₃) is made to flow;
- means (17) for generating UV ultraviolet rays in said sterilization chamber (15);
- a decontamination chamber (16) for decomposing ozone (O₃) into oxygen (O₂) downstream of the sterilization treatment in said sterilization chamber (15).

8. Apparatus according to claim 7, **characterized in that** it further comprises an infrared rays (IR) generator (18) associated with said sterilization chamber (15).

9. Apparatus according to claim 7, **characterized in that** it comprises a supervision and control electronic unit (13) that is active on said concentrator (11) and ozone generator (12), as well as for piloting said means (17) for generating UV ultraviolet rays which are interlocked to temperature, operating pressure and ozone (O₃) concentration sensors (19).

10. Apparatus according to claim 7, **characterized in that** it is configured with a receiving compartment or tank (23) container (20) that is closed by a watertight lid (21) that offers access to said sterilization chamber (15).

11. Apparatus according to claim 10, **characterized in that** said lid (21) is provided with lateral telescopic arms (22), having a pre-fixed friction, which allow keeping it angularly raised during the step of inserting and loading the objects (A) and items to be sterilized and sanitized.

12. Apparatus according to claim 11, **characterized in that** in the perimeter edge of the compartment (23) and of the lid (21) electromagnetic closure means (24) and counter-means (26) of the sterilization chamber (15) are provided.

13. Apparatus according to claim 12, **characterized in that** it comprises a supervision and control electronic unit (13) that is active on said electromagnetic closure means (24) and counter-means (26).

14. Apparatus according to claim 9, **characterized in that** it comprises a frontal control and display panel (29) of the touch screen type for said control unit (13).
